# EUROPÄISCHE PATENTSCHRIFT

(11) **EP 1 952 778 B1**
(45) Veröffentlichungstag und Bekanntmachung des Hinweises auf die Patenterteilung: **09.12.2009**
(21) Anmeldenummer: 07002066.4
(22) Anmeldetag: 31.01.2007
(51) Int. Cl.: A61B 19/00, G02B 5/122, A61B 17/00

(54) **Medizintechnischer Lasertarget-Marker und seine Verwendung**
Medical laser target marker and its use
Marqueur de cible de laser médical et son utilisation

(43) Veröffentlichungstag der Anmeldung: 06.08.2008
(73) Patentinhaber: BrainLAB AG, 85622 Feldkirchen (DE)
(72) Erfinder: Goldbach, Günter, 85457 Wörth/Wifling (DE)
(74) Vertreter: Rögner, Jürgen

(56) Entgegenhaltungen:
- WO-A-99/27837
- WO-A1-01/43654
- US-A1- 2006 058 604
- US-B1- 6 299 122

## Beschreibung

Die Erfindung betrifft einen medizintechnischen Lasertarget-Marker und seine Verwendung. Insbesondere betrifft die Erfindung das technische Gebiet der Kalibrierung bzw. Kalibrierungsprüfung für medizintechnische Trackingsysteme. Solche Trackingsysteme dienen dazu, die Raumposition von Trackingmarkern (zum Beispiel an medizinischen Instrumenten oder Apparaten bzw. an Patienten) zu ermitteln und medizintechnischen Navigationssystemen zur Verfügung zu stellen, um eine bildgeführte Chirurgie durchführen zu können. Da die Genauigkeit der Positionsbestimmung solcher Trackingsysteme von hoher Wichtigkeit für die korrekte Navigation ist, müssen die Systeme kalibriert werden; kalibrierte Systeme liefern exakte Positionsdaten für erfasste Trackingmarker. Ein Problem mit der Kalibrierung solcher Trackingsysteme liegt darin, dass sie vor Ort, also an den Einsatzorten im Krankenhaus bzw. in den Operationsräumen herkömmlicherweise nicht durchgeführt werden kann. Die Trackingsysteme müssen zu speziellen Prüfstellen verbracht werden, wo die Raumposition der Kalibrierungsmarker gegenüber installierten Trackingsystemen zur Überprüfung auch anderweitig bekannt ist. Außerdem müssen Trackingsysteme mit Trackingmarkern unterschiedlicher funktioneller Ausgestaltung (optisch-passive, optisch-aktive, magnetische Trackingmarker usw.) in eigens dafür vorgesehenen und unterschiedlichen Kalibrierungseinrichtungen kalibriert oder überprüft werden.

Aus der WO 01/43654 A1 sind ein Verfahren und eine Vorrichtung zur Instrumenten-, Knochensegment-, Gewebe- und Organnavigation bekannt, wobei ein Laserscanner verwendet wird, um die Form und Farbe von bestimmten Oberflächen, z.B. Markern zu erfassen. Ferner weist das neue Navigationssystem gemäß dieser Schrift eine stereoskopische Positionserfassungseinheit auf, welche dreidimensionale Koordinaten von 3D Markern ermitteln soll.

Aus der US 2006/058604 A1 ist ein Hybrid-Trackingsystem für die chirurgische Navigation bekannt, wobei vorgeschlagen wird, mehrere Tracking-Technologien während einer medizinischen Prozedur zu verwenden, wobei eine Abstimmungseinrichtung eine aktive Tracking-Technologie bestimmt.

Die US 6,299,122 B1 beschreibt einen Kantenadapter in Verwendung mit einem SMR-Element (spherically mounted retroreflector).

Es ist eine Aufgabe der vorliegenden Erfindung, die Kalibrierung bzw. die Überprüfung der Kalibrierung für solche Trackingsysteme zu vereinfachen, insbesondere sie vor Ort an der Einsatzstelle der Trackingsysteme zu ermöglichen. Diese Aufgabe wird erfindungsgemäß durch einen medizintechnischen Lasertarget-Marker gemäß dem Anspruch 1, eine Kalibrierungs- oder Kalibrierungsprüfungs-Vorrichtung gemäß dem Anspruch 7 bzw. durch die Verwendung eines Lasertarget-Markers gemäß dem Anspruch 8 gelöst. Die Unteransprüche definieren bevorzugte Ausführungsformen der Erfindung.

Ein erfindungsgemäßer medizintechnischer Lasertarget-Marker weist ein Positionsbestimmungs-Lasertarget, insbesondere ein SMR-Target (SMR= Spherically Mounted Retroreflector) und mindestens einen medizintechnischen optischen Trackingmarker einer ersten funktionellen Ausgestaltung auf. Ferner ist am Lasertarget-Marker gemäß der Erfindung mindestens ein weiterer medizintechnischer Trackingmarker einer anderen funktionellen Ausgestaltung angeordnet.

Im Folgenden soll zunächst auf die Lasertracking-Technik eingegangen werden, für die ein Lasertarget-Marker gemäß der vorliegenden Erfindung geeignet ist. Lasertrackingsysteme sind meßtechnische Systeme zur Bestimmung der Raumposition eines sogenannten "Targets". Das Lasertrackingsystem misst zwei Winkel und einen Abstand. Es weist das Target und eine Lasertracker-Positionsbestimmungseinheit (im Weiteren auch "Lasertracker") auf, und der Lasertracker sendet einen Laserstrahl zu dem retroreflektierenden Target, welches gegen das zu vermessende Objekt gehalten wird. Licht, das vom Target reflektiert wird, geht seine Bahn zurück und tritt wieder an derselben Position in den Tracker ein, wo es ihn verlassen hat. Retroreflektive Targets können unterschiedlich ausgestaltet sein, ein Beispiel ist das SMR-Target, das drei im 90°-Winkel zueinander ausgerichtete Reflektionsflächen aufweist und somit immer einen parallelen Strahl gleicher Lauflänge zurückschickt. Wenn das Laserlicht wieder in den Tracker eintritt, wird ein Teil davon in einen Interferometer gehen, welcher den Abstand zum SMR misst.

Es kann beispielsweise ein Helium-Neon-Laser verwendet werden, um den Weg zum Reflektor und zurück zum Interferometer zu messen. Es werden Winkelbestimmungseinrichtungen benützt, welche die Winkelausrichtung des Trackers an zwei mechanischen Achsen messen, der Azimut-Achse und der Elevations-Achse. Das Messen der Winkel und des Abstandes vom Interferometer genügt, um das Zentrum des SMR präzise zu lokalisieren. Eine Tracker-Software errechnet weiter die Verschiebung, die dem SMR-Radius entspricht und gelangt so zu den exakten Koordinaten der gescannten Oberfläche.

Die Abstandsbestimmung, die eine wichtige Funktion des Lasertrackers ist, kann entweder inkremental oder absolut sein. Eine inkrementale Abstandsmessung wird mit einem Interferometer und mit einem frequenzstabilisierten Helium-Neon-Laser durchgeführt. Das Laserlicht wird in zwei Strahlen aufgespalten; einer geht direkt zum Interferometer, der andere geht aus dem Tracker heraus, wird am SMR reflektiert und geht beim Rückweg wieder in den Interferometer hinein. In dem Interferometer interferieren die beiden Lichtstrahlen, was in einer Zyklus-Änderung resultiert, wenn der SMR seinen Abstand zum Tracker ändert, und zwar über eine Distanz, die einer halben Wellenlänge entspricht (etwa 0,3 Mikron). Elektronische Schaltungen zählen die Zyklus- oder Phasenänderungen und bestimmen den gemachten Weg.

Eine absolute Distanzmessung bestimmt den Abstand zum Target automatisch, sogar wenn der Strahl vorher unterbrochen worden ist. Infrarotlicht von einem Halbleiterlaser wird vom SMR reflektiert und geht zurück zum Tracker, wo es in ein elektrisches Signal umgewandelt wird. Elektronisch wird das Signal analysiert, um seine Laufzeit zu bestimmen, diesen Wert mit der Lichtgeschwindigkeit in Luft zu multiplizieren und den Abstand des Trackers vom SMR zu bestimmen. Im Rahmen der vorliegenden Erfindung können die beiden beschriebenen und alle sonstigen Arten von Lasertracker-Positionsvermessungen verwendet werden.

Es ist nun mit einem Lasertarget-Marker gemäß der Erfindung möglich, ein optisches Trackingsystem bezüglich der Kalibrierung zu prüfen oder zu kalibrieren, weil die Genauigkeit der Lasertracker-Messung vor Ort verwendet werden kann, um die Genauigkeit der Positionsbestimmung des Trackingsystems zu ermitteln. Ferner ist es mit einem medizintechnischen Lasertarget-Marker gemäß der vorliegenden Erfindung möglich, Trackingsysteme verschiedenster technischer Ausgestaltungen zu vermessen, zu kalibrieren oder zu überprüfen, weil mehrere funktionelle Arten von medizintechnischen Trackingmarkern an dem Lasertarget-Marker vorhanden sind.

Es können im Rahmen der Erfindung Trackingmarker von mehr als einer anderen funktionellen Ausgestaltung am Lasertarget-Marker vorhanden sein, und andererseits können von mindestens einer oder jeder funktionellen Ausgestaltung mehrere Trackingmarker vorhanden sein. Die Erfindung kombiniert also mindestens drei Tracking-Technologien in einem Target und gestattet es, die gewählte Anzahl von Messsystemen mit einer gemeinsamen Referenz zu kombinieren. Zusätzliche Targets wie RF-Antennen, Radar-Ortungseinrichtungen oder Ultraschallmarker können ebenfalls mit der Erfindung kombiniert werden, und ein typischer erfindungsgemäßer Targetmarker kombiniert die Systeme in einem Multi-Marker-Target auf dreidimensionaler oder sechsdimensionaler Basis. Die Erfindung macht somit den sehr genauen Lasertarget-Messstandard für die Überprüfung der Genauigkeit und Leistung anderer Trackingsysteme verfügbar.

Die Trackingmarker der ersten und/oder der anderen funktionellen Ausgestaltungen können aus Markergruppen, insbesondere aus Anordnungen mehrerer vordefiniert und charakteristisch zueinander angeordneter Marker bestehen. Eine erfindungsgemäße Idee besteht darin, eine geometrische Anordnung der unterschiedlichen Marker zu benutzen, bei welcher das Zentrum der Markerpositionen unterschiedlicher Marker (unterschiedlicher funktioneller Ausgestaltungen) das gleiche ist.

Gemäß einer erfindungsgemäßen Ausführungsform sind die Trackingmarker auf der Oberfläche des SMR-Targets angeordnet, die auch den SMR-Reflektor trägt.

Im Rahmen der vorliegenden Erfindung kann an dem Lasertarget-Marker mindestens ein Trackingmarker mindestens einer der folgenden funktionellen Ausgestaltungen angeordnet sein:
- optische Reflektions-Trackingmarker, insbesondere mit einem reflektierenden, speziell Infrarotstrahlung reflektierenden, Überzug, die beispielsweise als Kreisscheiben ausgestaltet sind;
- optische, aktiv abstrahlende Trackingmarker, die insbesondere Infrarotstrahlung abstrahlen, speziell LED-Trackingmarker;
- magnetische Trackingmarker, insbesondere Magnetspulen oder Magnetspulenanordnungen.

Die Erfindung betrifft gemäß einem weiteren Aspekt eine Kalibrierungs- oder Kalibrierungsprüfungs-Vorrichtung für ein medizintechnisches Trackingsystem, das ein optisches oder magnetisches Raumpositions-Detektionssystem, insbesondere ein stereoskopisches Kamerasystem oder ein Magnetortungssystem und eine Lasertracker-Positionsbestimmungseinheit umfasst, wie sie oben in verschiedenen Ausführungsformen beschrieben worden ist.

Ein weiterer Aspekt der vorliegenden Erfindung liegt in der Verwendung eines Lastertarget-Markers, der ein Positionsbestimmungs-Lasertarget, insbesondere ein SMR-Target (SMR = Spherically Mounted Retroreflector) und mindestens einen medizintechnischen Trackingmarker einer bestimmten funktionellen Ausgestaltung aufweist, als Kalibrierungs- oder Kalibrierungsprüfungs-Marker für ein medizintechnisches Trackingsystem. Die beschriebene Verwendung kann den Einsatz eines Lasertarget-Markers umfassen, wie er oben in verschiedenen Ausführungsformen beschrieben worden ist, und ebenfalls den Einsatz einer Kalibrierungs- oder Kalibrierungsprüfungsvorrichtung, wie sie im Vorhergehenden erwähnt worden ist.

Die Erfindung wird im Weiteren anhand einer bevorzugten Ausführungsform und mit Hilfe der beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: eine perspektivische Ansicht eines erfindungsgemäßen Lasertarget-Markers;
- Figur 2: den Lasertracker-Marker aus Figur 1 in einer Draufsicht und einer Seitenansicht; und
- Figur 3: eine schematische Darstellung eines erfindungsgemäßen Kalibrierungssystems für ein medizintechnisches Trackingsystem.

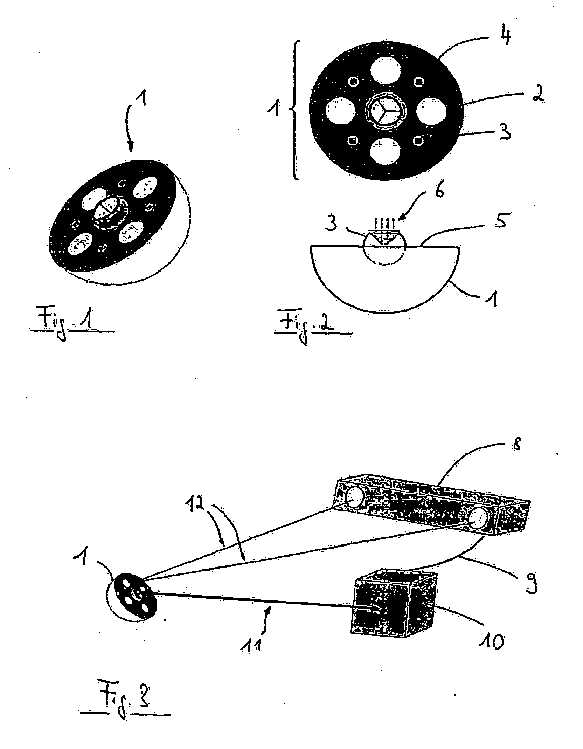

Mit Blick auf die Figuren 1 und 2 wird im Folgenden ein Multimarker-Lasertarget-Marker gemäß einer Ausführungsform der vorliegenden Erfindung beschrieben. Der Marker trägt insgesamt das Bezugszeichen 1 und hat im Wesentlichen eine Halbkugel-Form. Er trägt auf seiner kreisförmigen Schnittfläche 5 in der Mitte ein so genanntes SMR-Target 3, das eine nach oben vorstehende und ausgeschnittene Kugel umfasst, die drei zum Zentrum hin ausgenommene und senkrecht aufeinander stehende Reflektionsflächen aufweist. Durch diese Anordnung der Reflektionsflächen werden, wie durch das Bezugszeichen 6 dargestellt, eintreffende Licht-bzw. Laserstrahlen immer parallel zu ihrer Einfallsrichtung zurückgeworfen, und sie legen dabei auch in jedem Fall denselben Weg zurück, nämlich immer den Weg, der einer Reflektion am Kugelmittelpunkt des SMR-Targets entspricht. Mit diesem SMR-Target kann die hochgenaue Laser-Distanzmessung erfolgen, wie sie eingangs beschrieben wurde.

Zusätzlich sind auf dem Lasertarget-Marker 1 noch weitere Trackingmarkergruppen verschiedener funktioneller Ausgestaltungen vorhanden, nämlich eine Gruppe aus reflektierenden Kreisscheiben-Markern, von denen einer mit dem Bezugszeichen 2 gekennzeichnet ist, sowie eine Gruppe aus LEDs von denen eine wiederum mit dem Bezugszeichen 4 gekennzeichnet ist. Im vorliegenden Ausführungsbeispiel sind jeweils vier Marker für die beiden unterschiedlichen Trackingtechnologien vorgesehen, wobei das Zentrum der jeweiligen Marker-Anordnungen jeweils auch das Zentrum des SMR-Targets 3 ist. Bei erfindungsgemäßen Ausführungsformen könnten mindestens zwei Marker jeder Technologie vorhanden sein, insbesondere wieder mit dem Anordnungszentrum in der Mitte des Targets 3. Im vorliegenden Fall und bei einer speziellen erfindungsgemäßen Gestaltungsvariante liegen die Zentren der einzelnen Marker auf Kreisen um das Zentrum des SMR-Markers 3. Diese Gestaltungen gestatten es, bei der gleichzeitigen Erfassung des Lasertarget-Markers 1 durch ein Lasertarget-Positionsbestimmungssystem und ein medizintechnisches Trackingsystem, das Zentrum der Trackingmarker-Anordnungen zu bestimmen und unmittelbar mit der Lage des SMR-Target-Zentrums zu vergleichen.

In der Figur 3 wird schematisch eine typische Anordnung bzw. ein typisches Setup von zwei miteinander verbundenen Trackingsystemen gezeigt, die dasselbe Target verwenden, nämlich einen Lasertarget-Marker 1, wie er oben beschrieben worden ist. Eines der Trackingsysteme ist ein optisches, medizintechnisches Trackingsystem 8 und das zweite "Trackingsystem" ist die Lasertarget-Positionsbestimmungsvorrichtung 10, die einleitend auch als "Lasertracker" bezeichnet worden ist. Der Lasertracker 10 und das Trackingsystem 8 sind zum Datenabgleich miteinander verbunden, was durch eine Verbindungslinie 9 aufgezeigt ist. Die beiden Sichtlinien 12 zeigen, wie beispielhaft einer der LED-Marker 4 vom optischen Trackingsystem 8 positionell erfasst wird, und die Sichtlinie 11 zeigt den Strahlengang zwischen dem Lasertracker 10 und dem SMR-Target am Lasertarget-Marker 1.

Das videometrische Trackingsystem 8 (in diesem Fall ein stereoskopisches System) kann Beleuchtungseinrichtungen aufweisen (beispielsweise für die passiven Reflektionsmarker 2). Es misst die Raumposition des Zentrums des Targets 1 durch Triangulation über die Anmessung der vier Trackingmarker 4 und die Berechnung des Zentrums dieser Markeranordnung. Der Lasertracker 10 misst die 3D-Position (Raumposition) des SMR-Targets durch Winkelmessungen an seiner Lagerung und durch die Abstandsmessung, wie sie ebenfalls einleitend beschrieben worden ist.

Die Verbindung 9 zwischen Lasertracker 10 und Trackingsystem 8 kann verschiedene Ausgestaltungen annehmen, sie kann insbesondere von der Verbindungsart her kabellos oder unter Verwendung von Kabeln ausgeführt werden. Die Verbindung 9 kann auch für eine Synchronisierungseinrichtung stehen, die sicherstellt, dass die Messungen zum selben Zeitpunkt durchgeführt werden, was speziell dann wichtig ist, wenn das Target 1 bewegt wird und die Positionen verglichen werden müssen. Die Verbindung 9 kann auch eine starre mechanische Fixierung zwischen den beiden Einheiten umfassen, so dass die Relativposition zwischen dem Lasertracker 10 und dem optischen, stereoskopischen Trackingsystem 8 sich während der Messung nicht ändern kann.

Basierend auf dem oben beschriebenen Prinzip ist es nun möglich, die Messung eines der beiden Systeme zur Messung des anderen Systems in Referenz zu bringen. Dies macht es möglich, die Beschränkungen (Ungenauigkeiten) eines Systems zu überwinden sowie die Genauigkeit und Leistung eines Systems mit dem anderen zu vergleichen. Wenn das Target 1 bewegt wird, können der relative Abstand zwischen den beiden Messpunkten mit beiden Systemen ermittelt und Fehler erfasst und kompensiert werden.

Anders als videometrische Systeme bildet das Lasertracker-System einen anerkannten Messungs-Referenzstandard, der auf physikalische Einheiten zurückgeführt werden kann, und der Vergleich der videometrischen Messungen mit den Lasertracker-Messungen kann verwendet werden, um das videometrische System zu kalibrieren oder seine Genauigkeit bzw. Leistungsfähigkeit zu verifizieren, weil die Positionen, die vom Lasertracker-System ermittelt werden, mit den Messungen des videometrischen Systems direkt verglichen werden können.

Ferner ist es möglich, mit dieser Technik direkt die Genauigkeit zweier unterschiedlicher videometrischer Systeme mit einem (Lasertarget)Marker zu vergleichen. Hier ist ein präziser Leistungsvergleich möglich, weil viele Messunsicherheiten eliminiert werden können, die ansonsten auftreten würden, wenn verschiedene Trackingmarker für die unterschiedlichen Systeme eingesetzt würden.

Zusätzlich zu der Raumpositions-Messung des Multi-Marker-Targets 1 können auch sechs Freiheitsgrade berücksichtigt werden, wenn beispielsweise zwei weitere SMR-Targets auf dem Laser-Target-Marker angeordnet werden. Es ist dann möglich, nicht nur die dreidimensionale bzw. Raumposition des Target-Zentrums zu ermitteln sondern auch die Raumlage des Targets, die für Winkelgenauigkeits-Vergleiche von Interesse sein kann.

## Patentansprüche

1. Medizintechnischer Lasertarget-Marker (1), der ein Positionsbestimmungs-Lasertarget (3), insbesondere ein SMR-Target (SMR = Spherically Mounted Retroreflector), und mindestens einen medizintechnischen optischen Trackingmarker (2) einer ersten funktionellen Ausgestaltung aufweist, **dadurch gekennzeichnet, dass** am Lasertarget-Marker (1) mindestens ein weiterer medizintechnischer Trackingmarker (4) einer anderen funktionellen Ausgestaltung angeordnet ist, so dass mindestens drei Tracking-Technologien in dem medizintechnischen Lasertarget-Marker (1) kombiniert sind.

2. Lasertarget-Marker nach Anspruch 1, **dadurch gekennzeichnet, dass** Trackingmarker von mehr als einer anderen funktionellen Ausgestaltung vorhanden sind.

3. Lasertarget-Marker nach Anspruch 1 oder 2, **dadurch gekennzeichnet, dass** von mindestens einer oder jeder funktionellen Ausgestaltung mehrere Marker (2, 4) vorhanden sind.

4. Lasertarget-Marker nach einem der Ansprüche 1 bis 3, **dadurch gekennzeichnet, dass** die Trackingmarker (2, 4) der ersten und/oder der anderen funktionellen Ausgestaltung(en) aus Markergruppen, insbesondere aus Anordnungen mehrerer vordefiniert und charakteristisch zueinander angeordneter Marker bestehen.

5. Lasertarget-Marker nach einem der Ansprüche 1 bis 4, **dadurch gekennzeichnet, dass** die Trackingmarker auf der Oberfläche des SMR-Targets angeordnet sind, die auch den SMR-Reflektor (3) trägt.

6. Lasertarget-Marker nach einem der Ansprüche 1 bis 5, **dadurch gekennzeichnet, dass** mindestens ein Trackingmarker mindestens einer der folgenden funktionellen Ausgestaltungen an ihm angeordnet sind:
- optische Reflektions-Trackingmarker, insbesondere mit einem reflektierenden, speziell Infrarotstrahlung reflektierenden, Überzug, die beispielsweise als Kreisscheiben ausgestaltet sind;
- optische, aktiv abstrahlende Trackingmarker, die insbesondere Infrarotstrahlung abstrahlen, speziell LED-Trackingmarker;
- magnetische Trackingmarker, insbesondere Magnetspulen oder Magnetspulenanordnungen.

7. Kalibrierungs- oder Kalibrierungsprüfungs-Vorrichtung für ein medizintechnisches Trackingsystem, das ein optisches oder magnetisches Raumpositions-Detektionssystem, insbesondere ein stereoskopisches Kamerasystem (8), oder ein Magnetortungssystem und eine Lasertracker-Positionsbestimmungseinheit (10) umfasst, **dadurch gekennzeichnet, dass** sie ferner einen Lasertarget-Marker nach einem der Ansprüche 1 bis 6 umfasst.

8. Verwendung eines Lasertarget-Markers gemäß einem der Ansprüche 1 bis 6, der ein Positionsbestimmungs-Lasertarget (3), insbesondere ein SMR-Target (SMR = Spherically Mounted Retroreflector) und mindestens einen medizintechnischen Trackingmarker (2) einer bestimmten funktionellen Ausgestaltung aufweist, als Kalibrierungs- oder Kalibrierungsprüfungs-Marker für ein medizintechnisches Trackingsystem.

9. Verwendung nach Anspruch 8, mit einem Lasertarget-Marker (1) nach einem der Ansprüche 1 bis 6.

10. Verwendung nach Anspruch 8 oder 9, mit einer Kalibrierungs- oder Kalibrierungsprüfungs-Vorrichtung nach Anspruch 7.

## Claims

1. A medical laser target marker (1) comprising a position-determining laser target (3), in particular an SMR target (spherically mounted retro-reflector target) and at least one medical optical tracking marker (2) of a first functional configuration, **characterised in that** at least one other medical tracking marker (4) of a different functional configuration is arranged on the laser target marker (1), such that at least three tracking technologies are combined in the medical laser target marker (1).

2. The laser target marker according to claim 1, **characterised in that** tracking markers of more than one different functional configuration are provided.

3. The laser target marker according to claim 1 or 2, **characterised in that** a plurality of markers (2, 4) of at least one or of each functional configuration are provided.

4. The laser target marker according to any one of claims 1 to 3, **characterised in that** the tracking markers (2, 4) of the first and/or other functional configuration(s) consist of groups of markers, in particular arrangements of a plurality of markers which are arranged in a predefined and characteristic way with respect to each other.

5. The laser target marker according to any one of claims 1 to 4, **characterised in that** the tracking markers are arranged on the surface of the SMR target, wherein said surface also bears the SMR reflector (3).

6. The laser target marker according to any one of claims 1 to 5, **characterised in that** at least one tracking marker of at least one of the following functional configurations is arranged on it:
- optical reflection tracking markers, in particular comprising a reflective coating, especially a coating which reflects infrared radiation, which are for example configured as circular discs;
- optical, actively emitting tracking markers, which in particular emit infrared radiation, especially LED tracking markers;
- magnetic tracking markers, in particular magnetic coils or arrangements of magnetic coils.

7. A calibration or calibration testing device for a medical tracking system comprising an optical or magnetic spatial position detection system, in particular a stereoscopic camera system (8), or a magnetic localising system and a laser tracker position determining unit (10), **characterised in that** it also comprises a laser target marker according to any one of claims 1 to 6.

8. The use of a laser target marker in accordance with any one of claims 1 to 6 which comprises a position-determining laser target (3), in particular an SMR target (spherically mounted retro-reflector target) and at least one medical tracking marker (2) of a particular functional configuration, as a calibration or calibration testing marker for a medical tracking system.

9. The use according to claim 8, using a laser target marker (1) according to any one of claims 1 to 6.

10. The use according to claim 8 or 9, using a calibration or calibration testing device according to claim 7.

## Revendications

1. Marqueur de cible laser (1) médical qui comporte une cible laser (3) de détermination de position, en particulier une cible SMR (SMR = Spherically Mounted Retroflector) ainsi qu'au moins un marqueur de poursuite optique (2) médical d'une première forme de réalisation fonctionnelle, **caractérisé en ce que** sur le marqueur de cible laser (1) est disposé au moins un autre marqueur de poursuite (4) médical d'une autre forme de réalisation fonctionnelle, ce qui fait qu'au moins trois technologies de poursuite sont combinées dans le marqueur de cible laser (1) médical.

2. Marqueur de cible laser selon la revendication 1, **caractérisé en ce que** sont présents des marqueurs de poursuite de plus d'une autre forme de réalisation fonctionnelle.

3. Marqueur de cible laser selon l'une quelconque des revendications 1 ou 2, **caractérisé en ce que** sont présents plusieurs marqueurs (2, 4) d'au moins une ou de chaque forme de réalisation fonctionnelle.

4. Marqueur de cible laser selon l'une quelconque des revendications 1 à 3, **caractérisé en ce que** les marqueurs de poursuite (2, 4) de ou des autre(s) forme(s) de réalisation fonctionnelle sont constitués de groupes de marqueurs, en particulier d'agencements de plusieurs marqueurs disposés les uns par rapport aux autres de façon prédéfinie et caractéristique.

5. Marqueur de cible laser selon l'une quelconque des revendications 1 à 4, **caractérisé en ce que** les marqueurs de poursuite sont disposés sur la surface de la cible SMR qui porte aussi le réflecteur SMR (3).

6. Marqueur de cible laser selon l'une quelconque des revendications 1 à 5, **caractérisé en ce qu'**au moins un marqueur de poursuite d'au moins l'une des formes de réalisation fonctionnelle suivantes est disposé sur celui-ci :
- marqueurs de poursuite à réflexion optique, en particulier avec un revêtement réfléchissant, réfléchissant tout particulièrement le rayonnement infrarouge, qui sont conçus par exemple sous la forme de disques circulaires ;
- marqueurs de poursuite optiques rayonnant de manière active, qui rayonnent en particulier un rayonnement infrarouge, tout particulièrement des marqueurs de poursuite à LED ;
- marqueurs de poursuite magnétiques, en particulier bobines magnétiques ou agencements de bobines magnétiques.

7. Dispositif d'étalonnage ou de vérification d'étalonnage pour un système de poursuite médical qui comprend un système de détection de position spatiale optique ou magnétique, en particulier un système de caméra stéréoscopique (8), ou un système de localisation magnétique et une unité de détermination de position de marqueur laser (10), **caractérisé en ce qu'**il comprend en outre un marqueur de cible laser selon l'une quelconque des revendications 1 à 6.

8. Utilisation d'un marqueur de cible laser selon l'une des revendications 1 à 6, qui comporte une cible laser de détermination de position (3), en particulier une cible SMR (SMR = spherically Mounted Retroflector) ainsi qu'au moins un marqueur de poursuite optique médical (2) d'une forme de réalisation fonctionnelle déterminée, servant de marqueur d'étalonnage ou de marqueur de vérification d'étalonnage pour un système de poursuite médical.

9. Utilisation selon la revendication 8 avec un marqueur de cible laser (1) selon l'une quelconque des revendications 1 à 6.

10. Utilisation selon l'une quelconque des revendications 8 ou 9, avec un dispositif d'étalonnage ou de vérification d'étalonnage selon la revendication 7.
